# EUROPEAN PATENT SPECIFICATION

(11) **EP 2 337 607 B1**
(45) Date of publication and mention of the grant of the patent: **13.06.2018**
(21) Application number: 09737511.7
(22) Date of filing: 15.10.2009
(51) Int. Cl.: A61M 31/00, A61F 11/00

(54) **INNER EAR DRUG DELIVERY DEVICE**
VORRICHTUNG ZUR ABGABE VON ARZNEIMITTELN AN DAS INNENOHR
DISPOSITIF D ADMINISTRATION DE MÉDICAMENTS DANS L OREILLE INTERNE

(30) Priority: 15.10.2008 US 105493 P; 26.05.2009 US 180999 P
(43) Date of publication of application: 29.06.2011
(73) Proprietor: Med-El Elektromedizinische Geräte GmbH, 6020 Innsbruck (AT)
(72) Inventor: JOLLY, Claude, A-6020 Innsbruck (AT); MARTINI, Alessandro, I-35121 Podova (IT)
(74) Representative: Downing, Michael Philip
(86) International application number: PCT/US2009/060802
(87) International publication number: WO 2010/045432

(56) References cited:
- EP-A2- 1 424 050
- WO-A1-02/41666
- WO-A1-2005/072793
- WO-A2-01/82848
- WO-A2-02/102278
- US-A1- 2003 229 336
- US-A1- 2005 182 385

## Description

### FIELD OF THE INVENTION

The present invention relates to medical implants, and more specifically to a drug delivery device for the inner ear.

### BACKGROUND ART

There are many inner ear disorders which can lead to some degree of hearing loss. Among these are sudden hearing loss, noise induced hearing loss, progressive hearing loss, aminoglycoside induced hearing loss, presbiyacusis etc., autoimmune inner ear disorder, and infections (bacterial, viral, fungal). Many of the diseases that lead to partial or total hearing loss could utilize a therapeutic pharmaceutical treatment to reach some tissue or cells within the inner ear, for example, to arrest or reverse the hearing loss and improve hearing. Examples of therapeutic pharmaceutical molecules include without limitation cortico-steroids, peptides, and other proteins.

But there are relatively few ways to deliver therapeutic drugs to the inner ear. Typical clinical practice involves either oral, veinous, or arterial drug delivery. Topical drug delivery to treat the inner ear is limited to deposition of the drug at the round window and relying on diffusion of the drug through the round window to reach targeted cells. This may be accomplished by flooding the middle ear cavity with the drug in liquid form, or by applying a soaked sponge at or near the round window, for example, through an opening in the tympanic membrane. But a diffusion process through the round window is not particularly predictable or reliable. The permeability of the round window varies greatly between patients and by other criteria such as time of day, physical conditions, application methods, drug used. Thus, the amount of drug that reaches the inner ear through such delivery methods may vary anywhere between zero and toxically too much.

WO 2005/027293 discloses an implantable drug delivery apparatus for delivering a drug into a bodily fluid in a body cavity of a patient over a period of time, which includes a hollow member that defines at least one lumen for facilitating a unidirectional recirculating flow of a therapeutic fluid through the lumen.

### SUMMARY OF THE INVENTION

Embodiments of the present invention, which is defined in claim 1, are directed to a drug delivery device for the inner ear. A drug delivery member for the inner ear without any stimulation electrodes has an intra-cochlear portion penetrates into the inner ear of the patient and contains a drug eluting polymer material having at least one therapeutic drug which is released over time in a therapeutically effective amount into fluid in the inner ear of the patient.

In further specific embodiments, the intra-cochlear portion may have a cylindrical rod shape or a conical shape. The drug eluting polymer material may be a flexible polymer strip and/or a silicone material. The intra-cochlear portion may penetrate into the inner ear through the round window or through a cochleostomy opening. The therapeutic drug may be incorporated into the drug eluting polymer material and/or may be a coating on the surface of the drug eluting polymer material.

Some embodiments may also include a support wire within at least a portion of the drug delivery device to provide supporting stability to the drug delivery member. In such an embodiment, the support wire may further include a puncturing point towards an apical end of the intra-cochlear portion for puncturing the round window membrane to insert the intra-cochlear portion into the inner ear. In some embodiments, there may be an extra-cochlear portion that resides in the middle ear of a patient. The extra-cochlear portion may completely occlude where the intra-cochlear portion penetrates into the inner ear.

A method of delivering at least one therapeutic drug into the inner ear of a patient is also described. An intra-cochlear portion of a drug delivery member lacking stimulation electrodes and containing a drug eluting polymer material having at least one therapeutic drug is inserted through an opening into the inner ear. A therapeutically effective amount of the drug is then released from the drug eluting material over time into fluid in the inner ear of the patient.

In further specific embodiments, the intra-cochlear portion may be a cylindrical rod or have a conical shape. The drug eluting material may be a flexible polymer strip and/or a silicone material. The intra-cochlear portion may penetrate into the inner ear through the round window or a cochleostomy opening. The therapeutic drug may be incorporated into the drug eluting polymer material and/or may be a coating on the surface of the drug eluting polymer material.

In some embodiments, there may be at least a portion of the drug delivery member which contains a support wire to provide supporting stability to the drug delivery member. The support wire may include a puncturing point towards an apical end of the intra-cochlear portion to puncture the round window membrane for inserting the intra-cochlear portion into the inner ear. In some embodiments, there may be an extra-cochlear portion that resides in the middle ear of a patient. The extra-cochlear portion of the drug delivery member may completely occlude the opening into the inner ear. In some embodiments, the intra and extra cochlear portion of the drug delivery member may be substantially smaller than the round window membrane to avoid interference with the mechanical movement of the membrane. The intra-cochlear portion of the drug delivery member may include an anchor rod adapted to extend out into the middle ear, ending in a retrieval knob for pulling the drug delivery member out of the cochlea. For example, the anchor rod may be adapted to penetrate through the round window membrane. The intra-cochlear portion of the drug delivery member may have opposing conical ends.

Insertion of the intra-cochlear portion may be based on robotic surgery using a key hole approach. In addition or alternatively, inserting the intra-cochlear portion may use at least one of a suprameatal approach, an atticotomy approach, a trans-canal approach, a mastoidectomy and posterior tympanotomy approach, a tympano-meatal flap approach, and a myringothomy approach. A curved tubular or semi-tubular instrument may be useful to guide the drug delivery member into or toward the round window membrane.

Examples of a drug delivery system for delivering a therapeutic drug are also described. A drug delivery source may contain the therapeutic drug and be located adjacent to a mastoid cortex surface. An elongated delivery member defines a passage for carrying the therapeutic drug through the mastoid cortex surface into the middle ear.

The delivery member may include at least one delivery opening for delivering the therapeutic drug into the middle ear. In addition or alternatively, the delivery member may continue to a cochleostomy opening into a cochlea scala and there include at least one opening for delivering the therapeutic drug into the cochlea scala.

The delivery member may include a rigid section outside the cochleostomy opening and a flexible section inside the cochleostomy opening. The delivery member may be straight or curved. The delivery member may include an outer tube support that provides structural stiffening; for example, the outer tube support may be based on at least one of a metallic, polymer, and textile material. The delivery member may include an inner core support such as a rod element that provides structural stiffening.

### BRIEF DESCRIPTION OF THE DRAWINGS

Figure 1 shows anatomical structures of a typical human ear having a drug eluting rod in the inner ear according to one specific embodiment of the present invention.
Figure 2 shows a portion of another embodiment having an intra-cochlear section and an extra-cochlear section.
Figure 3 illustrates another embodiment which fully occludes the opening into the inner ear.
Figure 4 shows an example of an embodiment having an internal supporting wire.
Figure 5 illustrates an embodiment having a sharp metallic tip.
Figure 6 shows an example of an embodiment having an internal anchor rod and retrieval knob.
Figure 7 shows an embodiment as in Fig. 6 in position in the ear of a patient.
Figure 8 shows an exemplary drug delivery catheter not forming part of the present invention.

### DETAILED DESCRIPTION OF SPECIFIC EMBODIMENTS

Embodiments of the present invention are directed to a drug delivery device for insertion into the inner ear of a patient. Such a device is supported by recent advances in cochlear implant technology with regards to surgery and electrodes that preserve hearing. If proper surgical techniques and equipment are used, the inner ear can be entered either through the round window membrane or a cochleostomy without causing excessive trauma. It has been shown (both in animal testing and in human patient use) that appropriate placement of a cochlear implant electrode through the round window membrane over a limited distance into the scala tympani does not severely interfere with the mechanical functioning of the inner ear. Such drug delivery devices are introduced with minimum trauma through the round window membrane or cochleostomy.

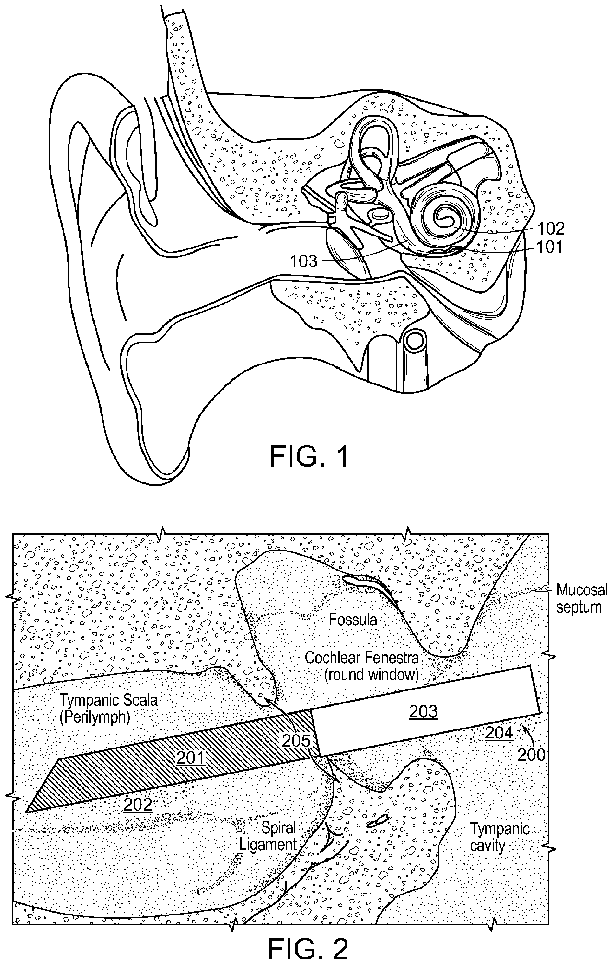

For example, as shown in Figure 1, an intra-cochlear portion of a drug delivery member **101** completely penetrates into the inner ear (cochlea) **102** of the patient and contains a drug eluting polymer material which is impregnated and/or coated with one or more therapeutic drugs (e.g., dexamethasone) that are released in therapeutically effective amounts over time directly into fluid in the inner ear of the patient. And unlike a cochlear implant electrode, there are no stimulation electrodes on the drug delivery member **101.** The intra-cochlear portion of the drug delivery member **101** and the drug eluting polymer material may be in the general shape of a cylindrical rod or conical shaped. Mechanisms for elution of the therapeutic drug from the drug eluting polymer material into an aqueous solution are known and reproducible. Different drug dosages can be achieved by varying the drug eluting polymer materials and their drug loading.

The drug delivery member **101** can be inserted into the inner ear **102** through a surgically created opening in the round window **103** or through a cochleostomy through the sidewall of the inner ear **102.** The drug delivery member **101** can be inserted through the round window **103** from the outer ear canal and a tympano-meatal flap, for example, using robotic surgery with a key hole approach. Alternatively, a suprameatal approach, an atticotomy approach, a trans-canal approach, a mastoidectomy and posterior tympanotomy approach, or a myringothomy approach for insertion of the drug delivery member **101.** When the round window **103** is not in a direct line with the tympanic membrane opening, a curved tubular or semi-tubular insertion instrument may be used to guide the drug delivery member **101** into or toward the round window **103.** The additional advantage of a curved insertion instrument is that it offers a rigid guide for a flexible drug delivery member **101** to be inserted into the inner ear through the round window **103.**

The drug delivery member **101** can remain permanently within the inner ear **102**-cochlear implant electrodes have been left in place for many years after initial implantation without complications. But in those cases where there is subsequently further hearing loss, the drug delivery member **101** can be removed (e.g., via the round window **10**3)**.** Then later another new drug delivery member **101** could be used, either with the same drug or with a more potent drug, with same concentration and dosage, or different concentration or dosage.

Figure 2 illustrates a portion of another embodiment of a drug delivery device **200** having a cylindrical rod-shaped intra-cochlear drug delivery member **201** having a diameter of 0.3 to 1.0 mm that is positioned within the cochlea **202** (inner ear), and another extra-cochlear section 203 that remains in the middle ear **204.** The intra-cochlear drug delivery member **201** is adapted to be inserted through the round window membrane **205** or through a cochleostomy drilled on the promontory. The insertion depth of the intra-cochlear drug delivery member **201** may be as great as 25 mm, but preferably is around 8 mm, which is the approximate length of the straight portion of the cochlea **202** before the intra-cochlear drug delivery member **201** would hit the outer wall of cochlea **202.** The intra-cochlear drug delivery member **201** and the extra-cochlear section **203** typically are substantially smaller than the round window membrane **205** to avoid interference with the mechanical movement of the membrane.

The intra-cochlear drug delivery member **201** that enters the cochlea **202** includes a drug eluting polymer material which includes a concentration of a therapeutic drug, either in the form of a surface coating or as particles or crystals that are mixed and interspersed within the polymer material so that the therapeutic drug can be slowly released from the intra-cochlear drug delivery member **201** over time in the surrounding aqueous solution (i.e., the inner ear fluids). The concentration loading of the therapeutic drug may be anywhere between 0.1 and 20% weight of drug particles in the drug eluting polymer material of the intra-cochlear drug delivery member **201.** Specific device arrangements and different drug loading and release profiles can be tailored to fit specific patient conditions and specific treatment requirements. Drug release can occur over a few weeks up to a several years depending on the drug loading and/or coating.

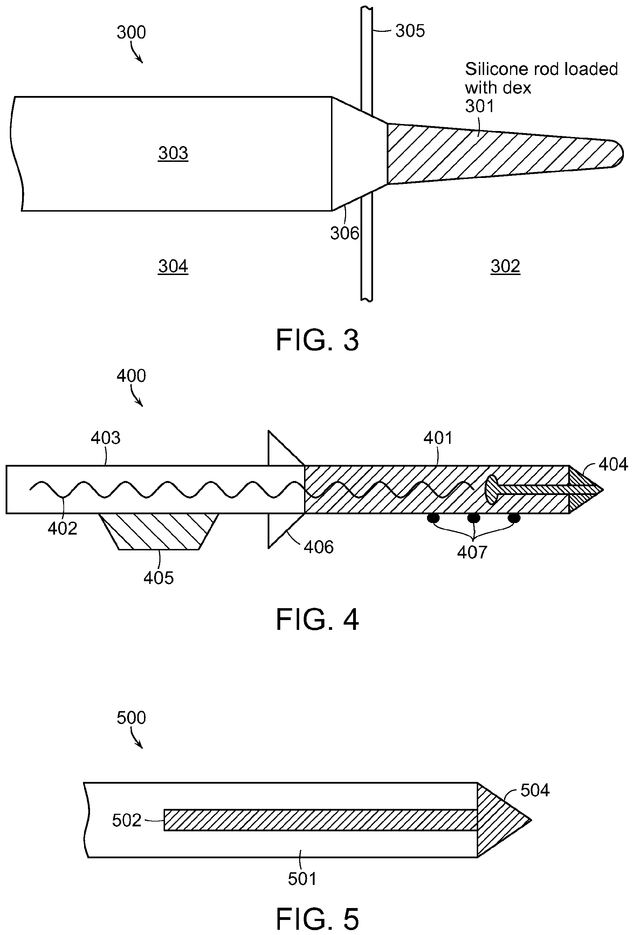

Figure 3 illustrates another embodiment of a drug delivery device **300** in the form of a generally cylindrical silicone polymer rod which fully occludes the opening into the cochlea **302.** The polymer rod has a tapered section **306** that when inserted fits snuggly in the opening to the round window membrane **305.** A tight fit minimizes fluid leakage from the inner ear **302** to the middle ear **304** and promotes rapid healing around the device at the round window membrane. Other embodiments may have a similar tapered section that would fit snuggly into a cochleostomy opening. An intra-cochlear drug delivery member **301** contains the therapeutic drug which is released over time into the inner ear fluids within the cochlea **302** The extra-cochlear portion **303** that remains in the middle ear **304** may be made of a non-eluting polymer material to avoid unwanted delivery of medicine to the middle ear **304.**

Figure 4 shows an example of an embodiment of a drug delivery device **400** having an internal supporting wire **402,** which acts to provide structural stiffening to provide sufficient rigidity to the drug delivery device **400.** In the embodiment shown the internal supporting wire **402** is wave-shaped, but which in other embodiments it may have other shapes, such as straight. One or more stabilizing holding wings **405** of the same polymer material as the rest of the drug delivery device **400** protrude from the extra-cochlear portion **403** to provide additional structural stability and aid in establishing a fixed position to the inserted device. A tapering section **406** occludes the opening in the round window membrane where the intra-cochlear portion **401** penetrates into the inner ear. Colored marking rings **407** around the intra-cochlear portion **401** give gradated information on the insertion depth of the device.

In the embodiment shown in Fig. 4, the internal supporting wire **402** extends from the extra-cochlear portion **403** to the intra-cochlear portion **401** and terminates in a sharp metallic puncturing point **404** towards the apical end of the intra-cochlear portion **401** for puncturing the round window membrane to insert the intra-cochlear portion **401** into the inner ear. In specific embodiments, this puncturing point **404** may be in the specific form of a coring or non-coring needle point. Assembling a beveled puncturing point **404** to a sufficiently flexible drug delivery device **400** can facilitate introduction of the device into the scala tympani in a single surgical move.

Figure 5 shows a partial cross-section of the intra-cochlear portion **501** of another embodiment of a drug delivery device **500** having a drug eluting silicone polymer material for time released delivery of a therapeutic drug. Metallic rod **502** provides mechanical stability and terminates in a sharp metallic tip **504.**

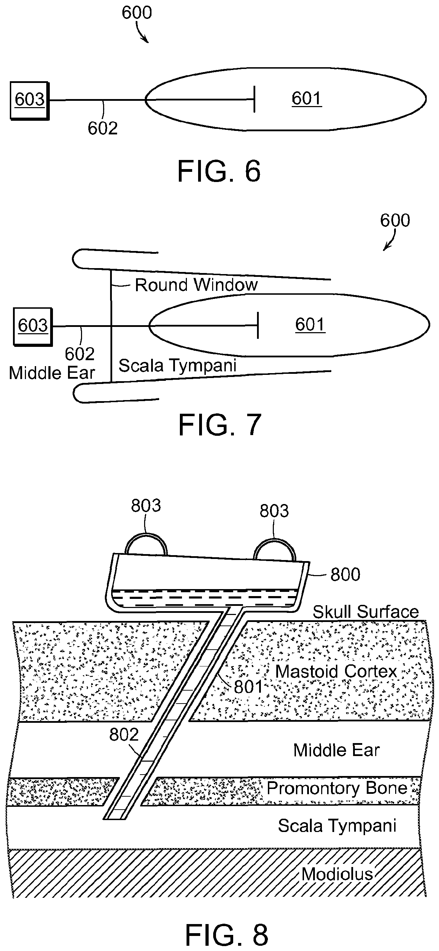

Figure 6 shows an example of an embodiment of another drug delivery device **600** where the intra-cochlear portion **601** has opposing conical ends. The conical-shaped ends of the intra-cochlear portion **601** facilitate insertion through a slit in the round window membrane as shown in Figure 7. In addition the intra-cochlear portion **601** has an anchored rod **602** in the interior of the drug delivery material, which extends out through the round window membrane into the middle ear. This allows healing of the penetration slit in the round window membrane slit the drug delivery material in the intra-cochlear portion **601** has been inserted completely into the scala tympani of the inner ear. Closure of the round window membrane is easier and faster around the thin rod of the anchor rod **602** instead of the larger diameter intra-cochlear portion **601** of the drug delivery member **600.** The smaller diameter rod shape of the anchor rod **602** also minimizes interference with the normal sound-induced vibration of the round window membrane. The end of the anchor rod **602** extending into the middle ear includes a retrieval knob **603** for pulling the drug delivery device **600** out of the cochlea as may sometimes be useful for replacing the drug delivery material or dealing with an infection. Retrieval of the drug delivery device **600** can occur after re-slitting the round window membrane around the anchor rod **602** which traverses the membrane. At that point, pulling back on the retrieval knob **603** allows the conical end of the intra-cochlear portion **601** to exit through the round window membrane toward the middle ear.

The techniques described herein also can be used as part of an exemplary drug delivery system for delivering a therapeutic drug to the middle ear or to the inner ear, which is not forming part of the invention. Figure 8 shows such an exemplary system where a drug reservoir **800** is implanted on the surface of the skull bone over a catheter passage **801** that passes through the mastoid cortex, the middle ear, an promontory bone directly into the cochlea scala. Instead of an implant electrode, a rigid catheter **802** delivers drug fluid from the drug reservoir 800 into the scala tympani. An example may be designed for delivering a drug to the middle ear, in which case, the entire drug catheter **802** may be rigid. For drug delver into the inner ear such as the scala tympani, then the apical tip of the drug catheter **802** is softer and flexible, similar in mechanical properties to the implant electrode described above. One or more septum ports **803** on the drug reservoir **800** may allow for insertion of a syringe needle to refill the reservoir.

It is understood that lubricants, lubricious coating, anti inflammatory coating, may be used in combination with the device and accessories described here. It is also understood that the implant electrode, drug delivery catheter, and the various accessories may be beneficial if using some type of endoral surgical approach, canal wall drill out, etc.

Although various exemplary embodiments of the invention have been disclosed, it should be apparent to those skilled in the art that various changes and modifications can be made which will achieve some of the advantages of the invention without departing from the true scope of the invention as defined by the appended claims.

## Claims

1. A drug delivery device for the inner ear, comprising:
a drug delivery member (101, 200, 300, 400, 500, 600) without stimulation electrodes and including an intra-cochlear portion (201, 301, 401, 501, 601) for penetrating into the inner ear of the patient,
**characterised in that**
the drug delivery member contains a drug eluting polymer material having at least one therapeutic drug for release over time in a therapeutically effective amount into fluid in the inner ear of the patient.

2. A device according to claim 1, wherein the intra-cochlear portion is a cylindrical rod (201).

3. A device according to claim 1, wherein the intra-cochlear portion has a conical shape (601).

4. A device according to claim 1, further comprising:
a support wire (402) within at least a portion of the drug delivery device to provide supporting stability to the drug delivery member.

5. A device according to claim 4, wherein the support wire (402) includes a puncturing point (404) towards an apical end of the intra-cochlear portion (401) for puncturing the round window membrane (103) to insert the intra-cochlear portion into the inner ear.

6. A device according to claim 1, further comprising:
an extra-cochlear portion (203, 303, 403) that resides in the middle ear of a patient.

7. A device according to claim 6, wherein the extra-cochlear portion completely occludes where the intra-cochlear portion penetrates into the inner ear.

8. A device according to claim 6, further comprising:
an anchor rod (502, 602) within the intra-cochlear portion (501, 601) of the drug delivery member and adapted to extend out into the middle ear, ending in a retrieval knob (603) for pulling the drug delivery member out of the cochlea.

9. A device according to claim 8, wherein the anchor rod is adapted to penetrate through the round window membrane.

10. A device according to claim 8, wherein the intra-cochlear portion of the drug delivery member has opposing conical ends.

## Patentansprüche

1. Vorrichtung zur Abgabe von Arzneimitteln an das Innenohr, umfassend:
ein Arzneimittelabgabeelement (101, 200, 300, 400, 500, 600) ohne Stimulationselektroden, und das einen intracochlearen Anteil (201, 301, 401, 501, 601) zum Eindringen in das Innenohr des Patienten einschließt,
**dadurch gekennzeichnet, dass** das Arzneimittelabgabeelement ein Arzneimittel eluierendes Polymermaterial mit mindestens einem therapeutischen Arzneimittel zur Freisetzung im Zeitverlauf in einer therapeutisch wirksamen Menge in Fluid im Innenohr des Patienten enthält.

2. Vorrichtung nach Anspruch 1, wobei der intracochleare Anteil ein zylindrischer Stab (201) ist.

3. Vorrichtung nach Anspruch 1, wobei der intracochleare Anteil eine konische Form (601) aufweist.

4. Vorrichtung nach Anspruch 1, ferner umfassend:
einen Haltedraht (402) innerhalb mindestens eines Anteils der Arzneimittelabgabevorrichtung, um dem Arzneimittelabgabeelement Haltestabilität bereitzustellen.

5. Vorrichtung nach Anspruch 4, wobei der Haltedraht (402) einen Durchstechpunkt (404) zu einem apikalen Ende des intracochlearen Anteils (401) zum Durchstechen der Membran des runden Fensters (103) einschließt, um den intracochlearen Anteil in das Innenohr einzusetzen.

6. Vorrichtung nach Anspruch 1, ferner umfassend:
einen extracochlearen Anteil (203, 303, 403), der im Mittelohr eines Patienten liegt.

7. Vorrichtung nach Anspruch 6, wobei der extracochleare Anteil dort vollständig okkludiert, wo der intracochleare Anteil in das Innenohr eindringt.

8. Vorrichtung nach Anspruch 6, ferner umfassend:
einen Ankerstab (502, 602) innerhalb des intracochlearen Anteils (501, 601) des Arzneimittelabgabeelements und vorgesehen, um sich in das Mittelohr auszustrecken, und der in einem Rückholknopf (603) endet, um das Arzneimittelabgabeelement aus der Cochlea ziehen zu können.

9. Vorrichtung nach Anspruch 8, wobei der Ankerstab vorgesehen ist, um durch die Membran des runden Fensters einzudringen.

10. Vorrichtung nach Anspruch 8, wobei der intracochleare Anteil des Arzneimittelabgabeelements gegenüberliegende konische Enden aufweist.

## Revendications

1. Dispositif d'administration de médicament pour l'oreille interne, comprenant :
un organe d'administration de médicament (101, 200, 300, 400, 500, 600) sans électrodes de stimulation et comprenant une partie intra-cochléaire (201, 301, 401, 501, 601) pour pénétrer dans l'oreille interne du patient,
**caractérisé en ce que**
l'organe d'administration de médicament contient un matériau polymère d'élution de médicament ayant au moins un médicament thérapeutique pour une libération dans le temps en une quantité thérapeutiquement efficace dans le fluide de l'oreille interne du patient.

2. Dispositif selon la revendication 1, la partie intra-cochléaire étant une tige cylindrique (201).

3. Dispositif selon la revendication 1, la partie intra-cochléaire ayant une forme conique (601).

4. Dispositif selon la revendication 1, comprenant en outre :
un fil de support (402) à l'intérieur d'au moins une partie du dispositif d'administration de médicament pour fournir une stabilité de support à l'organe d'administration de médicament.

5. Dispositif selon la revendication 4, le fil de support (402) comprenant un point de perforation (404) vers une extrémité apicale de la partie intra-cochléaire (401) pour perforer la membrane de fenêtre ronde (103) pour insérer la partie intra-cochléaire dans l'oreille interne.

6. Dispositif selon la revendication 1, comprenant en outre :
une partie extra-cochléaire (203, 303, 403) qui réside dans l'oreille moyenne d'un patient.

7. Dispositif selon la revendication 6, la partie extra-cochléaire obstruant complètement l'endroit où la partie intra-cochléaire pénètre dans l'oreille interne.

8. Dispositif selon la revendication 6, comprenant en outre :
une tige d'ancrage (502, 602) à l'intérieur de la partie intra-cochléaire (501, 601) de l'organe d'administration de médicament, adaptée pour s'étendre dans l'oreille moyenne, se terminant en un bouton de récupération (603), et destinée à retirer l'organe d'administration de médicament hors de la cochlée.

9. Dispositif selon la revendication 8, la tige d'ancrage étant adaptée pour pénétrer à travers la membrane de fenêtre ronde.

10. Dispositif selon la revendication 8, la partie intra-cochléaire de l'organe d'administration de médicament présentant des extrémités coniques opposées.
